# EUROPEAN PATENT SPECIFICATION

(11) **EP 2 178 680 B1**
(45) Date of publication and mention of the grant of the patent: **07.05.2014**
(21) Application number: 08788962.2
(22) Date of filing: 30.07.2008
(51) Int. Cl.: B25J 9/00, A61B 5/00, A61F 5/01

(54) **WEARABLE MECHATRONIC DEVICE**
TRAGBARES MECHATRONISCHES GERÄT
DISPOSITIF MÉCATRONIQUE PORTABLE

(30) Priority: 30.07.2007 IT PI20070088; 10.08.2007 IT PI20070102
(43) Date of publication of application: 28.04.2010
(73) Proprietor: Scuola Superiore Di Studi Universitari S. Anna, 56127 Pisa (IT)
(72) Inventor: CATTIN, Emanuele, I-56020 Santa Maria a Monte (PI) (IT); VITIELLO, Nicola, I-56025 Pontedera (PI) (IT); GIOVACCHINI, Francesco, I-56121 Pisa (IT); VECCHI, Fabrizio, I-56023 Cascina (PI) (IT); CARROZZA, Maria Chiara, I-56100 Pisa (IT); ROCCELLA, Stefano, I-57127 Livorno (IT)
(74) Representative: Celestino, Marco
(86) International application number: PCT/IB2008/001990
(87) International publication number: WO 2009/016478

(56) References cited:
- EP-A- 1 260 201
- EP-A- 1 437 108
- WO-A-95/10396
- WO-A-2007/029419
- DE-A1- 10 313 747
- FR-A- 2 845 592
- JP-A- 7 204 233
- US-A- 4 665 900
- US-A- 5 631 861
- US-A- 6 110 130
- US-A1- 2006 052 732

## Description

### Field of the invention

The present invention relates to a wearable mechatronic exoskeleton for rehabilitation of a patient's limb, in particular for the elbow joint, but also for shoulder, wrist or knee, etc. and for sports training. Further applications concern the possibility of using the exoskeleton for biomechanical studies of the upper limb.

In addition the present invention relates to an hand wearable mechatronic exoskeleton for orthopedic and neurological rehabilitation of the hand. Further applications concern the possibility of using the exoskeleton for the control and handling of a remote device (teleoperation) and for biomechanical studies of the hand.

In particular, the invention relates to a combined hand-elbow exoskeleton.

### Description of the prior art.

Among mechatronic systems used for limb rehabilitation, in particular for the upper limb, some of them are exoskeleton type devices. Some of them have a transmission of the motion to the exoskeleton joints by means of mechatronic exoskeleton devices with force feedback, used in different fields like tele-operation, virtual reality, etc.

US2004106881 describes a wearable rehabilitation system for the elbow actuated by cables in agonist-antagonist configuration. Different types of remote actuators can be used to pull the actuation cables. Actuation is made using a single cable wrapped around the actuation pulley and driven by one actuator. This solution doesn't allows to control separately the position and the stiffness of the joint: at least two actuators are required. The kinematic coupling between the system and the elbow of the human arm is a hinge joint, which is unable to self-aligning to the arm joint. Mechanical interfaces for upper limb coupling are made of sleeves made of closed-cell or open-cell polymeric foam fixed to the arm by straps. This well known device doesn't use an elastic sensorized double shell system.

Another well-know system is the one described by WO9740789, allowing to align a revolute joint with the starting angular inclination of the axis of the joint. This alignment is fixed along all the range of motion of the joint. Main disadvantage of this system is the missing self alignment of the revolute joint during human arm joint motion. The mechanical interface for the limb coupling is obtained using a shell made of plastic material with polymeric foam with straps to fix the limb. Furthermore, no actuation is provided by the system.

Other mechatronic exoskeleton devices exist with force feedback directly applied on the hand, for different applications like teleoperation, virtual reality, etc.

For instance, WO9510396 describes an actuated hand exoskeleton, where actuation is obtained by means of tendons wrapped on pulleys. This actuation allows only the extension of the fingers since it is designed to simulate a contact between the inner part of the hand and a virtual object. So, this device is not adapted to be employed for hand rehabilitation that requires a control of both opening and closing movements of the hand then of an agonist-antagonist scheme of actuation. Other disadvantages of the said device are the consistent mass of the system on the upper side of the hand, both on the fingers and on the hand back, due to the mechanical structure of the exoskeleton and to the actuation groups directly assembled on the device. In addition, such device requires a glove to be worn that prevents some movements of the hand and a contact between the object and the skin of the hand.

Another well known device is described in US3967321. It is applied on the thumb and on the index and middle finger coupled together and it is driven by means of a Bowden cable. The device allows only the flexion of index and middle fingers of the metacarpophalangeal articulations to allow the grasping of an object between the aforesaid fingers and the thumb. Return of the finger in extended position is provided by a spring, so, also this device has the disadvantages of not providing an agonist-antagonist actuation.

Other devices are known, for instance an actuated hand exoskeleton, described in "Occupational and physical therapy Using a hand exoskeleton based exerciser", Proceedings of 2004 IEEE/RSJ International Conference on Intelligent Robots and Systems, 2004, Sendai, Japan, di Sarakoglou, I.; Tsagarakis, N.G.; Caldwell, D.G, where forces are generated by electrical motor and transmitted to the joints by means of tension cables. This exoskeleton has the disadvantage that the user has to wear a glove, where the mechanical part of the device that allows to apply the forces on the fingers is located.

Other actuated hand exoskeletons exist, for example that described in "Development and Control of a Hand Exoskeleton for Rehabilitation of Hand Injuries", IEEE/RSJ Int. Conf. on Intelligent Robots and Systems 2005, of A. Wege, K. Kondak, and G. Hommel. This paper concerns a one-finger exoskeleton provided with 4 Degrees of Freedom (3 DoFs in flexion-extension movements and 1 DoF in abduction-adduction movements). This device has the disadvantage to have quite bulky mechanical parts on the upper side of the finger; these parts are provided by a pulley and Bowden cables transmission connected with a remote actuation system that allows to move the finger. Regarding the sensors assembled on the device, Hall effect sensors are placed on each joint to measure the angular position of the finger and resistance force sensors are placed on every interface between the phalanxes and the mechanical part of the device.

Patent document EP 1260201 A discloses a wearable mechatronic device according to the preamble of claim 1.

### Summary of the invention

An object of the present invention is to provide a mechatronic exoskeleton for limb rehabilitation, adapted to transmit the forces and torques necessary to move the limb without undesired stresses applied on the limb joints.

Another object of the invention is to provide a mechatronic exoskeleton for limb rehabilitation, adapted to properly follow the anatomical joint axis and a proper transmission of the driving torque applied to the limb joint that avoids undesired stresses due to misalignment between anatomical joint axis and the joint axis itself.

A further object of the invention is to provide a mechatronic exoskeleton for limb rehabilitation, adapted to transmit a driving torque to the limb using an agonist-antagonist actuation scheme. This solution allows to implement bioinspired control systems adapted to interact in the best way with the patient's limb during the rehabilitation phases.

Another object of the invention is to provide a mechatronic exoskeleton for limb rehabilitation, adapted to easily adapt to the anthropometric dimensions of the different users by means of an ad-hoc interface that is customized for each user.

It is still an object of the invention to provide a mechatronic exoskeleton for limb rehabilitation that is wearable and with low encumbrance and weight.

Another object of the invention is to provide a mechatronic exoskeleton for limb rehabilitation allowing a correct coupling with human limb impedance.

Likewise, it is an object of the present invention to provide a mechatronic device provided with an actuated hand exoskeleton that allows fulfilling controlled motion of the finger of the hand aimed at a therapeutic rehabilitation of the hand.

It is, also, an object of the present invention to provide a mechatronic device provided with an actuated hand exoskeleton with low encumbrance both on the lateral side of the fingers and on upper and lower side of the hand to allow an easy wearability.

It is a further object of the present invention to provide a mechatronic device provided with an actuated hand exoskeleton that allows free movements of all the phalanxes of the hand.

An additional object of the present invention is to provide a mechatronic device provided with an actuated hand exoskeleton that allows the kinematic compatibility between the movements of the fingers and the movements permitted by the exoskeleton.

It is another additional particular object of the present invention to provide a mechatronic device provided with an actuated hand exoskeleton with adjustable size to be adaptable to different hands.

It is, finally, an object of the present invention to provide a mechatronic device provided with an actuated hand exoskeleton that allows to control the stiffness at the joints.

These and other objects are achieved by a wearable mechatronic device for limb rehabilitation, said limb including a proximal element and a distal element connected by a joint, said joint having an instantaneous joint axis, including.
- a proximal stiff bearing structure attachable to said proximal element of said limb and a distal stiff bearing structure attachable to said distal element of said limb, said proximal and distal structures pivotally connected about a revolute axis;
- means to generate a driving torque between said proximal and distal structures;
- means to measure the angular position between said proximal and distal structures;
said device characterized in that it includes instantaneous self-aligning means for aligning said revolute axis with said instantaneous joint axis, said instantaneous self-aligning means including means for moving selectively said revolute axis around a point, inside a space defined by two opposite cones having vertex coincident on said point.

In particular, said self-aligning means includes a couple of opposite adjustable joints, each adjustable joint being formed by a ring and a shaft that is the shaft of said revolute axis, said ring and said shaft being blocked from relatively rotating about said revolute axis, but being adapted to rotate in a limited way around two axes perpendicular to said revolute axis, such that a conical motion of said shaft with respect to said point is defined.

Preferably, each shaft that is linked between said proximal and distal structures, pivotally receives a driven pulley integral to the other among said proximal and distal structures.

In other words, the shaft is constrained to said proximal structure and the driven pulley is integral to said distal structure, or vice versa.

In this way by operating said pulley, said revolute axis follows operatively said instantaneous joint axis without undesired stresses induced on the articulation. So, such a device, by passively self-aligning to orientation changes of the instantaneous joint axis of the limb, transmits a torque between said proximal and distal elements oriented along the joint axis direction, which is the physiological axis. Hence, using this solution it is possible to transmit to the elbow joint a pure torque avoiding undesired force components stress the user's limb in an inappropriate way.

Advantageously, said opposite adjustable joints are respectively connected with a first and a second kinematic chain placed specularly to each other with respect to a longitudinal plane of the limb and connected with said proximal or distal structure.

In particular, each kinematic chain has in series:
- a rigid link having a first end slidingly connected to said adjustable joint by means of a circular guide and a second end connected to said proximal or distal structure;
- at least one connecting rod hinged at an end to said rigid link close to said second end and at another end to a rocker arm;
- said rocker arm being centrally hinged to said proximal or distal structure and to one end of said connecting rod, the other end of said rocker arm being hinged to the connecting rod of the other kinematic chain.

In this way, said adjustable joint allows to orient the connection shafts, which are sized to receive a driving torque necessary to move the joint. The device is designed and sized so that the axis defined by the circular guides is perpendicular and incident with the axis of the connection shafts. The rocker arm system ensures an anti-symmetric coupling between the two links to ensure keeping the alignment of the connection shaft axes.

In particular, said connecting rods have adjustable length and said connecting rods have preferably a ball- and-socket joint connection with said rigid link or said rocker arm.

Advantageously, said linear and/or circular guides are coupled to low friction sliders, in particular ball sliderss.

In particular, said rigid link is L shaped and bent along a substantially cylindrical surface so that it is operatively displaced around the limb.

Advantageously, said proximal and distal structures have an anatomical shape, substantially saddle-like shaped with U section, to allow to the limb to be housed within them; in particular, said structures including mechanical stops to avoid over-extension of the limb. Such conformation gives to the device, according to the invention, a small encumbrance.

Advantageously, said proximal and distal structures have a hollow box-like shape, in particular made of carbon fiber material. This way, the device according to the invention is light, and the air space of the aforesaid external structure can be used for passing wiring of sensors and actuation systems, but also to house the support mechanical structures.

Advantageously, said means for generating a driving torque between said first and second structure includes actuating means in agonist-antagonist configuration.

Advantageously, said actuating means in agonist-antagonist configuration includes:
- a pulley connected with said adjustable shaft, said pulley being integral to said proximal or distal structure;
- a couple of cables that respectively wrap and unwrap on said pulley to operate a rotating motion between said proximal structure and said distal structure, said cables having an end constrained respectively to a first and a second peripheral connection point of said pulley.

Advantageously, said cables are sliding inside of a respective sheath, said sheath having an end constrained to said distal structure. This way, the displacement of the pulley with respect to the connection point of the Bowden cables allows to discharge the reaction forces of the sheaths thereof on the structure on which the pulley is constrained, making the structure of the device self-balanced.

Alternately, instead of an idle pulley, said actuating means includes a planetary reduction gear having:
- a wrapping pulley for said actuation cables;
- a sun gear coaxially integral to said wrapping pulley;
- planetary gears that mesh with said sun gear;
- a carrier integral to said proximal or distal structures;
- an internal gear slidingly connected with said shaft of said adjustable joint.

Advantageously, said self-aligning shaft includes a torque sensor adapted to measure the torque between said proximal structure and said distal structure.

In particular, said torque sensor is integrated on said pulley, said pulley having a plurality of spokes, said spokes having at least one strain gauge adapted to transmit force information to a control system, said at least one strain gauge being preferably put in a Wheastone bridge to filter the force components applied by the cables owing to the pure torque between the structures.

Alternately, means are provided to measure the forces applied to each of said actuation cables, including a load cell having a fixed first end and a second end pivotally connected with a deflection pulley on which said cable is operatively connected, said cable pressing transversally on said deflection pulley and operating said load cell, said load cell giving as output a signal proportional to force transverse component Fy of said force, said force being obtainable by said force transverse component Fy.

The use of a Bowden cable transmission system allows to displace the actuation system remotely with respect to the user. This way, it is possible to use different types of actuators to operate the system.

Advantageously, said proximal and distal structures comprise each an internal shell and an external shell, connected by a coupling means, such as an elastic connection means including at least one elastic bushing with spherical joint to allow a limited relative motion, in particular including a sensor means and wherein the internal shell is operatively in touch with the distal or proximal structure of the limb, while said external shell coupled to said internal shell. In particular, said proximal or distal internal shell is selected from the group comprised of:
- an external layer of thermoforming material, in particular polypropylene;
- an internal padding layer of low thickness expanded polymer material, in particular plastazote or evalux.

In other words, the external layer is thermoformed on the user's limb shape and the used material (polypropylene) has the characteristics to be stiff enough to support a mechanical connection which is elastic enough to adapt to the limb in a coupling position. The materials used as internal padding are thin and with low deformability and they maintain the user's comfort_because they are thermoformed on the user's limb. The use of materials for orthosis assures a correct transpiration and the space between the two shells provides an appropriate air circulation for draining moisture. The use of an internal shell thermoformed on the limb allows to distribute forces on the coupling surface with the patient's limb, avoiding force concentration on local areas of the limb that could damage the skin of the patient's arm and generate pain and discomfort.

Advantageously, the actuated hand exoskeleton is further provided for a user's hand, said hand having a metacarpus from which fingers extend formed by proximal phalanxes, medial phalanxes, distal phalanxes and including various articulations among which metacarpal phalangeal articulations (MCP) and abduction-adduction and flexion-extension articulations between said metacarpus and said proximal phalanxes, flexion-extension interphalangeal proximal articulations (PIP) between the proximal phalanxes and the medial phalanxes, and interphalangeal distal articulations (DIP) between the medial phalanxes and the distal phalanxes, said exoskeleton including:
- an external backing element applicable on the metacarpus;
- shell-like elements applicable on each phalanx, said shell-like elements being connected in series to each other starting from said backing element by means of respective joints;
- means to arrange operatively and automatically said joints in coincidence of each respective articulation of said hand.

In particular, said means to arrange said joints comprises:
- three operated and controlled revolute couplings corresponding to said flexion-extension MCP, PIP, DIP articulations of said hand;
- two longitudinal sliding couplings corresponding to the MCP and PIP articulations of said hand;
- a passive revolute coupling corresponding to said abduction-adduction articulation of said hand.

Preferably, said sliding couplings are passive.

In particular, the sliding coupling corresponding to said MCP articulation is placed between said MCP rotating coupling and said PIP rotating coupling; likewise, the sliding coupling corresponding to said PIP articulation is placed between said PIP rotating coupling and said DIP rotating coupling.

In particular, each actuated revolute coupling comprises:
- two pulleys placed on the opposite side of said shell-like elements integral to said shell;
- a Bowden cable for each pulley, having a first end connected to said pulley and a second end connected to an actuation group, said cable causing the rotation of the pulley when actuated and hence the rotation of the joint to which it is connected.

Preferably, said Bowden cables are housed in grooves made on lateral sides of said shell type elements.

Advantageously, said actuated revolute couplings include respective position sensors adapted to measure in real time the hand posture.

In particular said backing element and each shell-like element are made of stiff material with low thickness.

In particular, said backing element and each shell-like element are lined internally with compliant orthopedic material.

Advantageously, said shell-like elements are coupled to said phalanxes by means of elastic open ring clip.

Alternately, said shell type elements are coupled to said phalanxes by means of fastening straps.

According to another aspect of the invention, a combined hand-elbow exoskeleton is provided formed by said actuated hand exoskeleton and said mechatronic device for limb rehabilitation.

### Brief description of the drawings

The invention will be made clearer with the following description of some exemplary embodiments, exemplifying but not limitative, with reference to the attached drawings wherein::
- figures 1 and 2 show two different perspective views of a proximal bearing structure connected to a bearing distal structure by a self-adjusting joint of a wearable mechatronic device according to the invention; such structures are without internal shell to simplify the description;
- figure 3 shows a detailed view of the wearable mechatronic device, equipped with an angular position sensor;
- figure 4 shows a perspective view of the wearable mechatronic device;
- figure 5 shows a particular of a self-adjusting joint according to the invention, including a driving pulley on which a first and a second Bowden cable respectively wrap and unwrap;
- figures 6,7 and 8 show how the mechatronic device can be applied to a user's upper limb, in particular to the arm and the forearm, covering the rear side of the limb;
- figure 9 shows an example of an internal shell shaped on the user's limb for improving the user's comfort, having elastic and sensorized connection elements to the stiff structure of the mechatronic device;
- figure 10 shows in detail the mechanical structure of the self-aligning joint according to the invention, having a revolute axis moving inside a double cone, adapted to follow the articulation axis between the

arm and forearm of the user;
- figure 11 shows a kinematic scheme of the mechanical structure;
- figure 12 shows a diagrammatic cross section of a possible embodiment of a self-aligning joint according to the invention;
- figure 13 shows in perspective view such self-aligning joint;
- figures 14 and 15 show schematically a embodiment of force sensor means applied to the drive cables to measure the torque applied to the joints;
- figure 16 shows with a perspective view of an actuated hand exoskeleton according to the invention, in particular with four fingers;
- figure 17 shows a perspective view of the exoskeleton, having one finger in closed position;
- figures 18 and 19 show respectively two side views of opposite sides of the same finger of the exoskeleton;
- figures 20 and 21 show respectively two lateral views of opposite sides of the same finger where also the end of the Bowden cables are shown that move the phalanxes of the finger acting on the respective pulleys with which they engage;
- figure 22 shows a longitudinal section of a finger of such exoskeleton;
- figure 23 shows a sectional view of the finger of the exoskeleton, worn by the user's finger;
- figure 24 shows a kinematic scheme reproducing the model of a single finger of the hand exoskeleton according to the invention;
- figure 25 shows a side view of a finger of the exoskeleton according to the invention to which the kinematic scheme of figure 24 has been superimposed to facilitate understanding;
- figure 26 shows a combined hand-elbow exoskeleton according to the invention.

### Description of the preferred embodiments

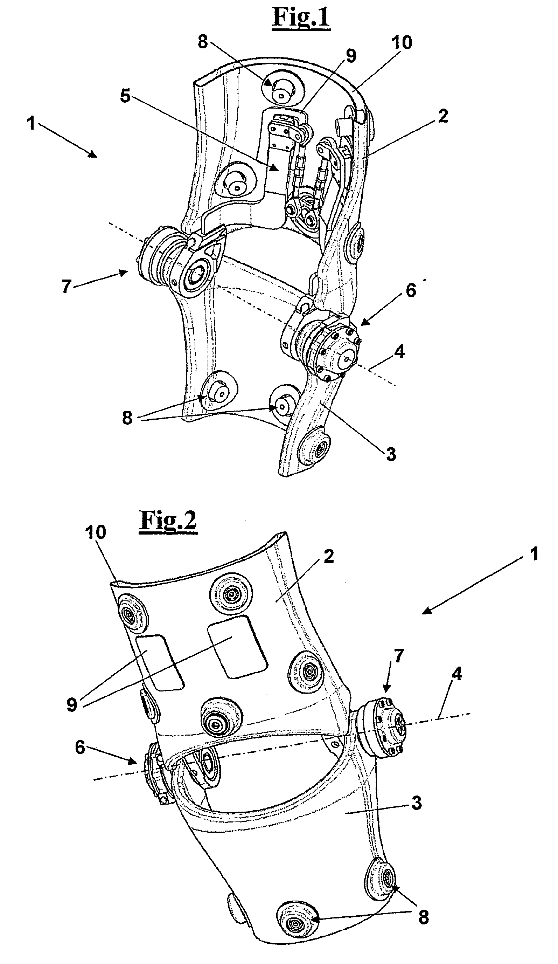

In figures 1 and 2 a possible embodiment is shown of a wearable mechatronic device for the rehabilitation of a patient's limb. In particular, the embodiment described in the following figures is referred to an actuated orthosis for the upper limb, for elbow rehabilitation, but, obviously the same principles can be extended and applied to other joints, such as the shoulder and wrist joints, or to joints of the lower limb.

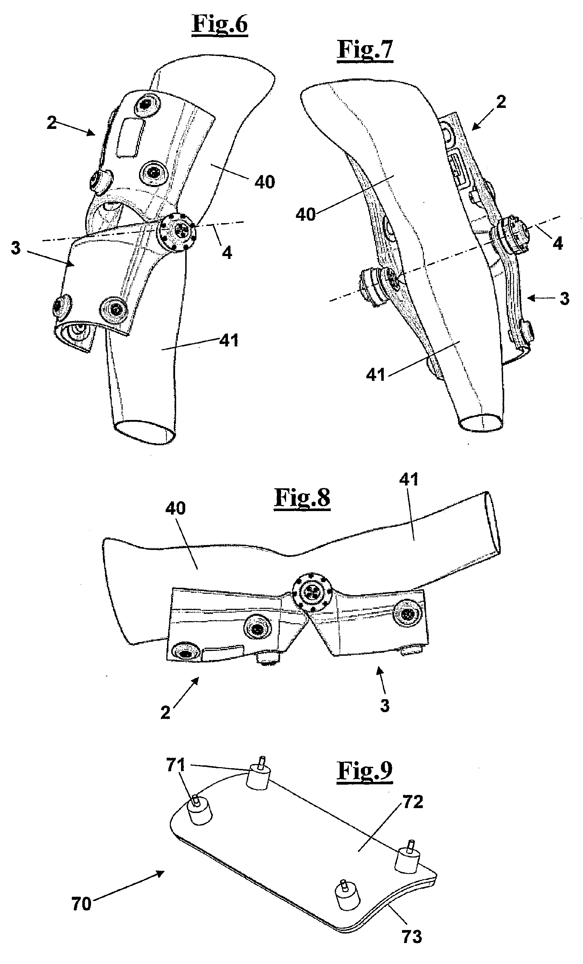

The device described in figures 1 and 2 includes a stiff proximal structure 2, attachable for instance to an arm 40, as shown in figures 6, 7 and 8, and a stiff distal structure 2, attachable for instance to a forearm 41. The two structures 2 and 3 are connected by a self-aligning revolute joint 6 and 7 that enables them to rotate about a self-aligning revolute axis 4, in order to follow instantaneously the articulation axis between the arm 40 and the forearm 41. Indeed, such rotation, in a human upper limb is not simply a rotation about a fixed axis but around an axis that varies its position during the rotation itself.

The proximal and distal structures 2 and 3, which bear the forces involved with the articulation movements, comprise two hollow double walled external shells, having inside an air space 10, and made for instance of carbon fiber. The shape of the shells is bound to the anatomic shape of the articulation, and is designed to reduce the weight of the device, as well as to avoid over-extension of the arm by using mechanical stops not shown in the figures.

Shells 2 and 3 are hollow and double walled to maintain a lightweight structure, and also for using the air gap 10 for passing the wirings of the sensors and of the actuation system not shown in figures 1 and 2. Furthermore, the space between the internal and the external shells is designed for housing self-aligning means 5 that supports the adjustable joints 6 and 7 and is designed to follow the orientation of the axis of the elbow during its movement. This mechanism is adapted to passively self-align to the changes of the direction of an articulation axis including the possibility to transmit a torque along the direction of the axis of the joint itself 6 and 7. Using this solution, it is possible to transmit to the elbow joint a pure torque avoiding that undesired components load in an improper way the patient's limb.

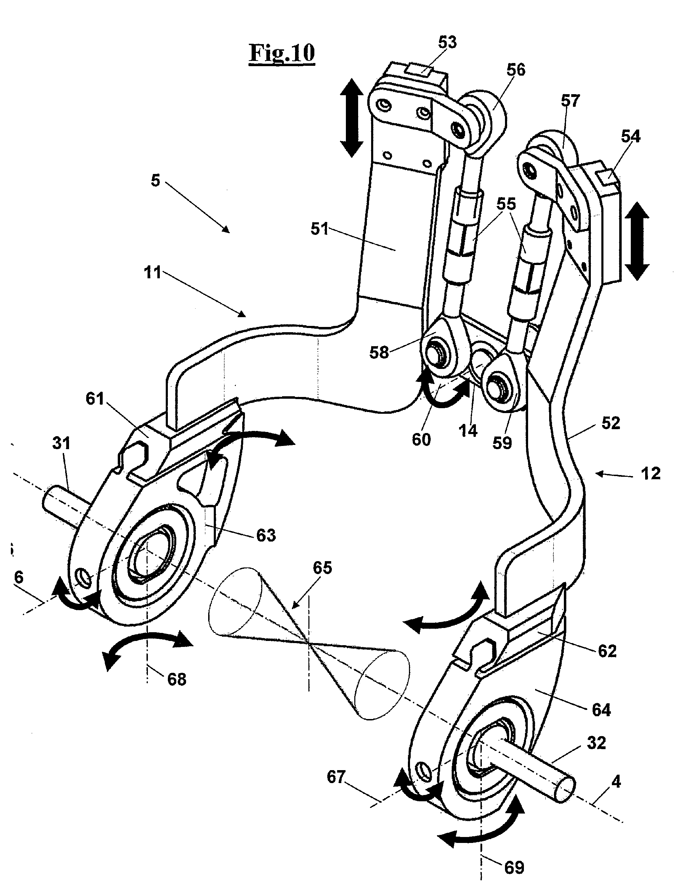

As shown in figure 10, the aforesaid self-aligning means 5 of self-aligning axis 4 includes two kinematic chains 11 an 12, which are specular to each other and have an end connected with a rocker arm 60 hinged on an intermediate point to the proximal structure. The kinematic chains 11 and 12 are connected to rocker arm 60 by two respective connecting rods 55, each of them having ends connected by ball and socket joints 56, 58 and 57, 59. The kinematic chains 11 and 12 slide parallel to each other, sliding on respective linear guides 53 and 54 integral to the proximal structure. Such linear guides 53 and 54 can be, for instance, low friction ball bearing linear guides. Each kinematic chain 11 and 12 includes a rigid link 51 or 52, having a first end connected to the linear guide 53 or 54 and to the ball and socket joint 56 or 57 of the connecting rod 55, and having a second end with a circular guide 61 or 62, for instance a ball circular guide, with its centre matching the centre of the articulation. The circular guides 61 and 62 drive a respective two Dof joint 63 and 64, which allows to orient two respective connection shafts 31 and 32, which are adapted to be the driving torque required to move the articulation.

The system is designed and dimensioned such that the axis identified by the circular guides 61 and 62 is perpendicular and incident to the axis defined by the connection shafts 31 and 32. The rocker arm system 60 guarantees an anti-symmetric coupling between the two links 51 and 52 to ensure an alignment of the axes of connection shafts 31 and 32. The rotation axis 4 of the joint can passively move within double cone 65, shown in figure 10. Kinematic chains 11 and 12 are assembled within the proximal structure and are shaped in order to move freely.

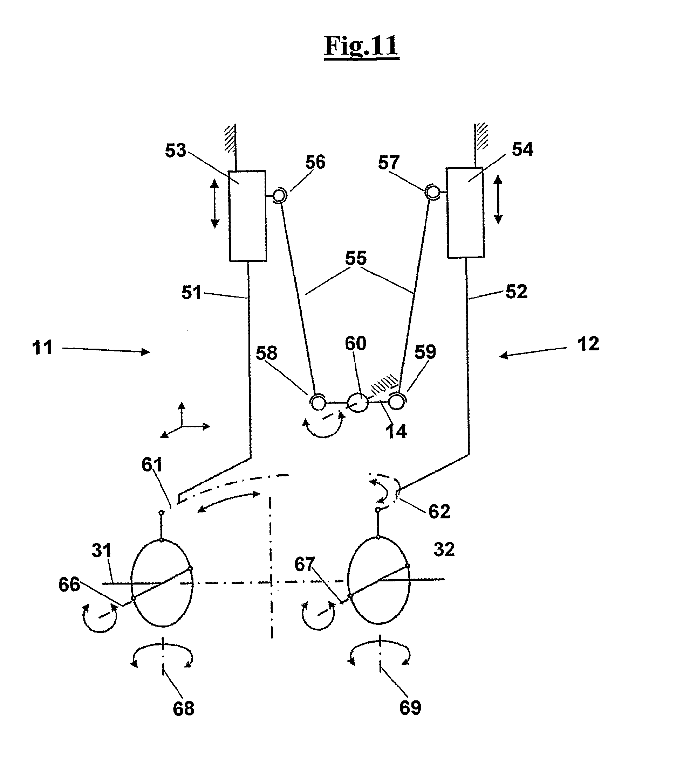

A kinematic scheme of the self-aligning means 5 of figure 10 is described in figure 11, where are distinguished the specular kinematic chains 11 and 12 connected by rocker arm 60 hinged on revolute joint 14 to the proximal structure, working as a frame. At the ends of rocker arm 60, connecting rods 55 are constrained by means of ball and socket joints 58 and 59. Connecting rods 55 are also connected to an end of links 51 and 52 by means of ball and socket joints 56 and 57. The same ends of links 51 and 52 are connected to the frame by means of respective low friction ball linear guides 53 and 54. At the opposite ends of links 51 and 52 a respective sliding circular joint 61 and 62 is connected having a circular guide where a respective adjustable joint slides. Each of the two adjustable joints has two DoFs and form the perpendicular revolute joints 66,68 and 67,69, allowing to the connection shafts to move freely along the direction of the rotation axis between the user's arm and forearm.

The kinematics of the mechanism is designed so that possible external stresses acting on the shells are discharged mainly on rocker arm 60. In this way the user's limb is not stressed, for instance, by the weight of the structure and by the dynamic stresses that can be generated during motion, for instance, those generated by centrifugal forces.

In one of the possible embodiments the device according to the invention includes an inner shell 70, shown in fig. 9, which is operatively in contact with the limb and is coupled with the bearing structure. In this case the self-aligning means 5 are placed between the shell and the structure, which is U-shaped in order to allow the insertion of the limb within the structure. The shell 70 is shaped anatomically, as is thermoformed on the limb, allowing to distribute the stresses transmitted by the elastic elements on all the patient's limb coupling surface, avoiding concentration of stresses in local areas of the limb that could stress the tissue of the patient's arm generating pain and uncomfortable feelings. The shell is therefore a comfortable mechanical interface between the external rigid bearing structure 2 and 3 and the limb of the user, avoiding that the user's limb interferes with the self-aligning means.

The inner shell is made by layers: it has an external layer made by a thermoforming material 72, for instance polypropylene, and an inner layer 73 made by thermoforming low thickness expanded polymeric materials, for example plastazote or evalux.

Such materials are known from a consolidate experience in the orthopedic field and so they guarantee an appropriate biocompatibility.

The external shell is thermoformed on the shape of the user's limb and the used material (polypropylene) is stiff enough to support a mechanical connection and is elastic enough to adapt to the limb.

Between the stiff structures 2 and 3 and the inner shell there are elastic connection means that include at least a bushing with a spherical joint. In the case described in the figure there are four elastic elements 71 to connect the rigid structures 2 and 3 of figures 1, 2, 6-8, while the rigid structures have corresponding connection bushings 8. The elastic elements 71 can be equipped with a three-axis force sensor adapted to measure the force vector transmitted through them. The measure of the interface force values allows to guarantee an appropriate comfort for the user and to obtain additional information on the distribution of the driving torque that actuates the joint.

Due to the elastic connection between the rigid structure 2 and 3 and the shell 70, the rigid structure 2 and 3, within certain limits, can balance the possible misalignments due to soft tissue artefacts (muscles, fat, skin, etc).

Furthermore, use of an inner shell that is shaped on the anatomy of the limb of the user avoids adjustments in situ of the structure of the system, to simplify the wearing procedure. Each user will be provided with an own set of inner shells that will be coupled to the rigid structures by means of specifically arranged elastic elements.

All the above described figures show only the external rigid structure to simplify the description, but it is clear that the structure is coupled to shell 70. Obviously, embodiments of the present invention without shell are possible.

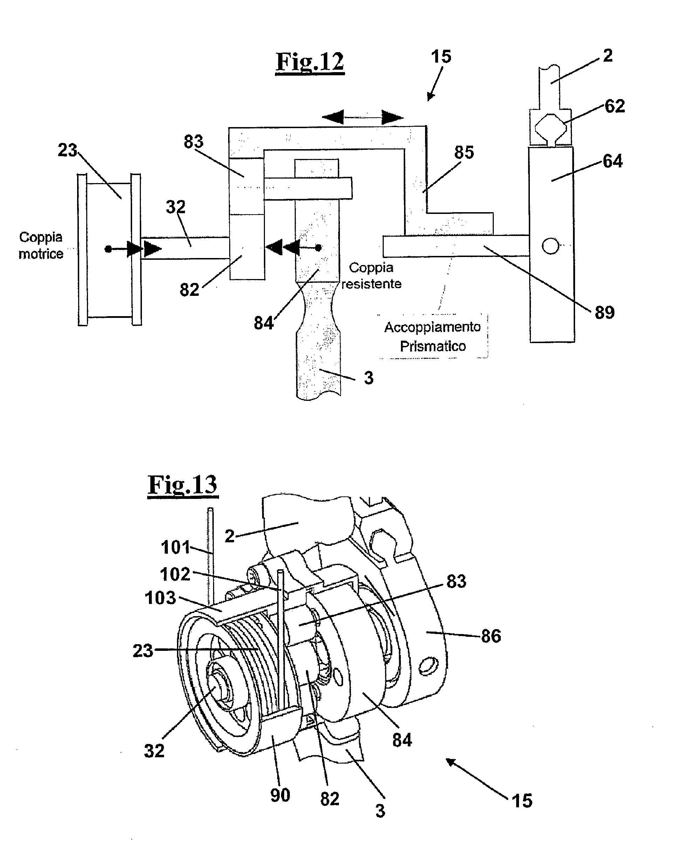

Figure 12 shows a cross sectional view of an embodiment of motion transmission means between proximal structure and distal structure 2 and 3, while figure 13 shows such transmission means in a perspective view. In this case, such transmission means includes a pulley 23 on which actuation cables 101 and 102 wrap, a sun gear 82 integral to shaft 32 and to pulley 23, planetary gears 83, for example 4, of the reduction planetary gear, a carrier 84 integral to distal shell 3, an inner gear 85 of the reduction planetary gear sliding on shaft 89 of the two DoFs joint.

The transmission of the rotation motion to the distal shell 3 is obtained by the above mentioned planetary reduction gear, with the object of reducing the value of the incoming torque necessary for the motion. This solution allows to reduce the forces transmitted to cables 101 and 102 and to driving pulley 23.

The cables are placed on a "capstan" like smooth pulley 23. A calibrated spacer 90 is put between the groove of pulley 23 and the wall of crown 103 to prevent an inappropriate wrapping of cables 101 and 102. The ends of cables 101 and 102 are fixed to the pulley 23 in diametrically opposite positions, as well as the antagonist cable 101 on the surface of the pulley and vice-versa.

Pulley 23 is coaxial to pinion 82, which is the input sun gear for the driving torque in the planetary gear reducer. The carrier 84, integral to the external distal shell 3, support the planetary gears 83 of the reducer. The motion of the carrier 84 therefore generates the relative rotation of two external shells 2 and 3. A sliding joint between inner gear 85 and shaft 89, made by a grooved profile, guarantees the transmission of only the torque of inner gear 85 to shaft 89 of the two DoFs the joint connected to the external proximal shell 2.

In this way the reaction torque by operating on the inner gear, is transmitted on the proximal external shell, allowing the rotation of the carrier 84 and at the same time a translation with respect to the two DoFs joint.

The physiological rotation angle of the user's elbow is less than 180°, therefore also the planetary reduction gear is designed to guarantee a rotation angle of carrier 84 less than 180°. On this basis, inner gear 85 is provided with a slot, obtained by partial milling the external wall of inner gear 85, to enable distal external shell 2 integral to carrier 84 to move.

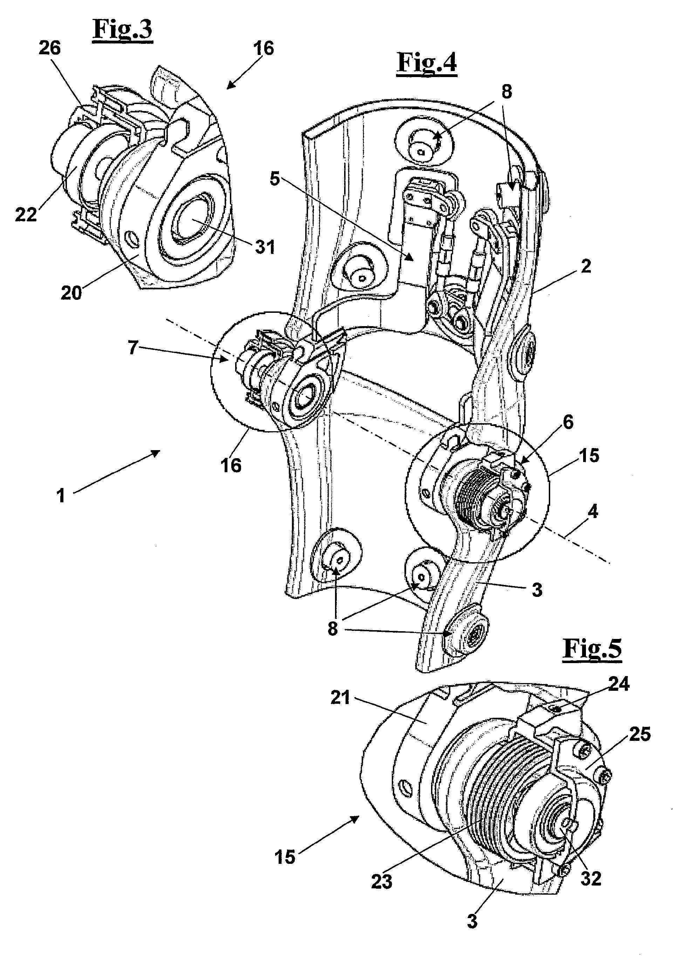

An alternative embodiment of the adjustable joint is shown in figures 3, 4 and 5. In this case, it provides a transmission cable with sheaths (Bowden cable), not shown, connected to the ends in agonist-antagonist scheme. The two agonist and antagonist cables, not shown, respectively wrap and unwrap on the pulley 23 which is integral to the distal rigid structure. The placement of pulley 23 with respect to connection point 24 of the Bowden cables allows to discharge the reaction forces of the sheath on the structure where driving pulley 23 is placed, obtaining a self-balanced external distal structure. The pulley is designed to be a torque sensor in order to transmit the torque information to the control system. In particular, the spokes of the pulley, not shown, are equipped with not shown strain gauges, conveniently placed according to a Wheatstone bridge scheme in order to filter the components of the tension forces of the cables, obtaining only the pure torque information.

Pulley 23 is designed to be coupled by means of a sliding joint with connection shaft 32 or 31 of the adjustable joint to maintain the axis 4 of the joint aligned with the one of the articulation of the limb of the user.

The Bowden cable transmission allows to place the actuation system upstream of the wearable mechatronic device in a remote position with respect to the user, without discharging weights on the user same. In this way it is possible to provide several types of actuators to move the system.

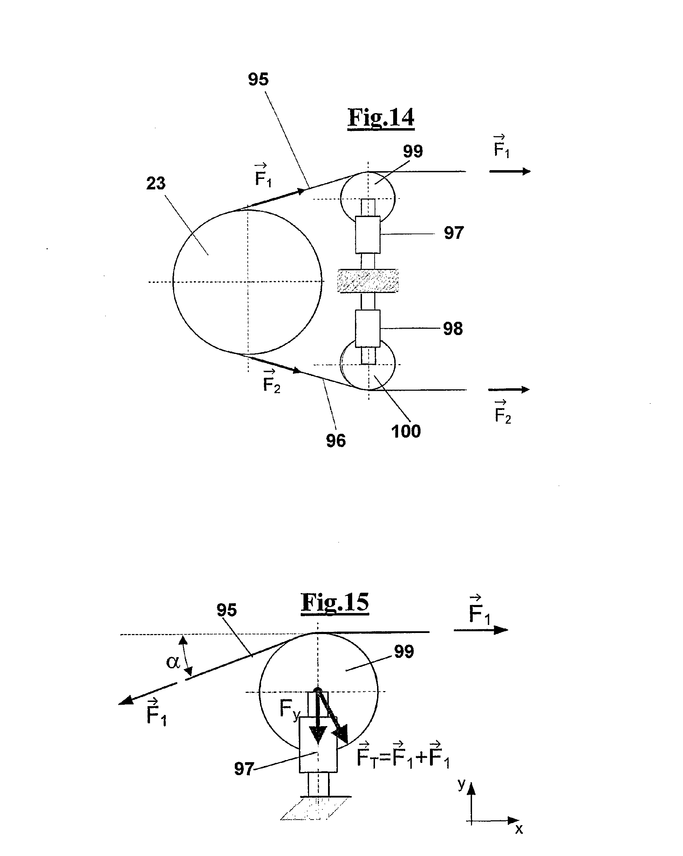

As shown in figures 14 and 15, means can be provided for measuring the forces applied on the actuation cables to move the joints. In said figures the agonist antagonist actuation cables 95 and 96 are shown, engaging with pulley 23, integral to either one of the distal or proximal shell.

The means to measure the forces for each of the two cables 95 and 96, include a load cell 97 and 98, to measure a transverse component Fy of the resultant force FT applied on the cables, where the load cells 97 and 98 have a free end connection to a respective deflection cable pulley 99 and 100.

The cable 95 coming from the driving pulley 23, where it is wrapped, is deflected by the pulley 99 by a known angle □. The pin, not shown, around that is rotating the pulley 99 is connected to a load cell 97 adapted to measure a transverse component Fy of the resultant force acting on the pulley. Known the angle α it is possible by means of trigonometric computation to calculate the value of the force F applied on the cable. The load cells 97 and 98 are designed to reduce the encumbrance. The same measurement principle is applied on tension gauges for cables.

The knowledge of the values of the forces applied on the cables allows to compute in indirect way the value of the applied torque.

The described features assist using to the wearable mechatronic device as active orthosis for upper limb rehabilitation and personal assistance.

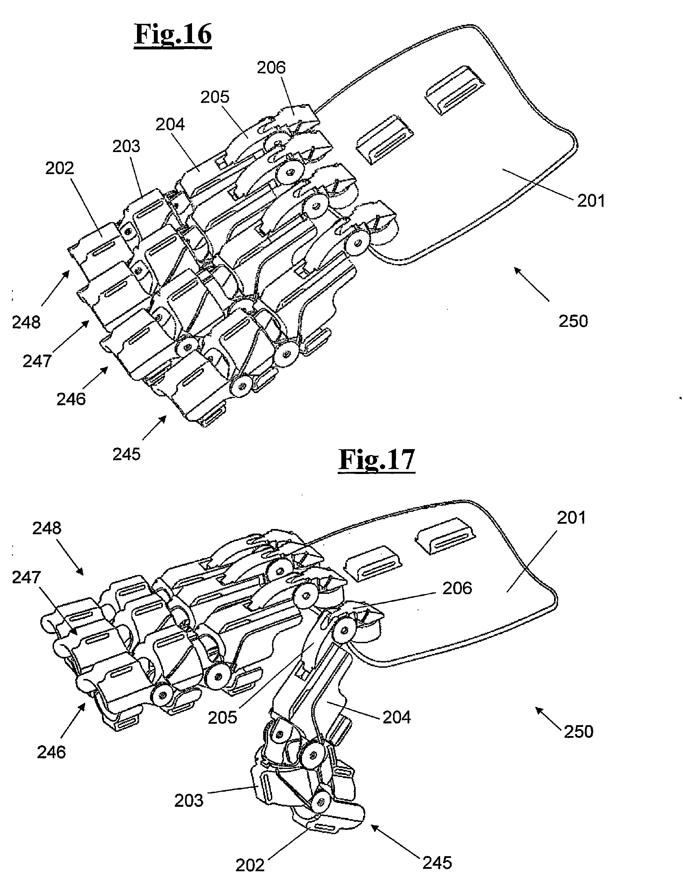

According to the present invention a wearable mechatronic device can further comprise an hand exoskeleton 250 for a user's hand as shown in figure 16. The hand exoskeleton 250 has four independent exoskeleton fingers 245, 246, 247 and 248 that extend form a backing element 201 externally coupled on the metacarpus and adapted to connect the system to the hand.

Obviously, depending on the needs, it is possible to make an exoskeleton 250 with a different number of fingers, for instance with five fingers adding the thumb to be applied to the whole hand, or alternatively with less than four fingers when it has to be applied only to a part of the hand.

Similarly, figure 17 shows such exoskeleton according to the invention with finger 245 in a bent position.

Each exoskeleton finger 245, 246, 247 and 248 has a series of shell type elements 202, 203, 204 with low thickness and coupled to them. Such shell type elements 202, 203, 204 are coated on their inner side by compliant materials.

A coincidence with the anatomical axis of the finger skeleton and the axis of the system is maintained, according to the invention, also during the flexion-extension movements of the fingers, that, as well known moves the articulation axes 242, 243 and 244 to enable rotation of the finger bones.

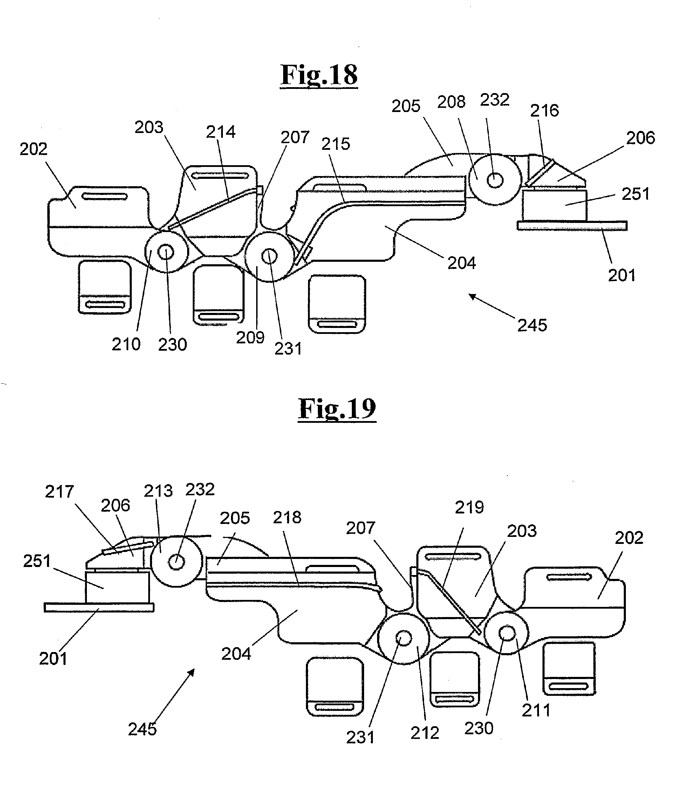

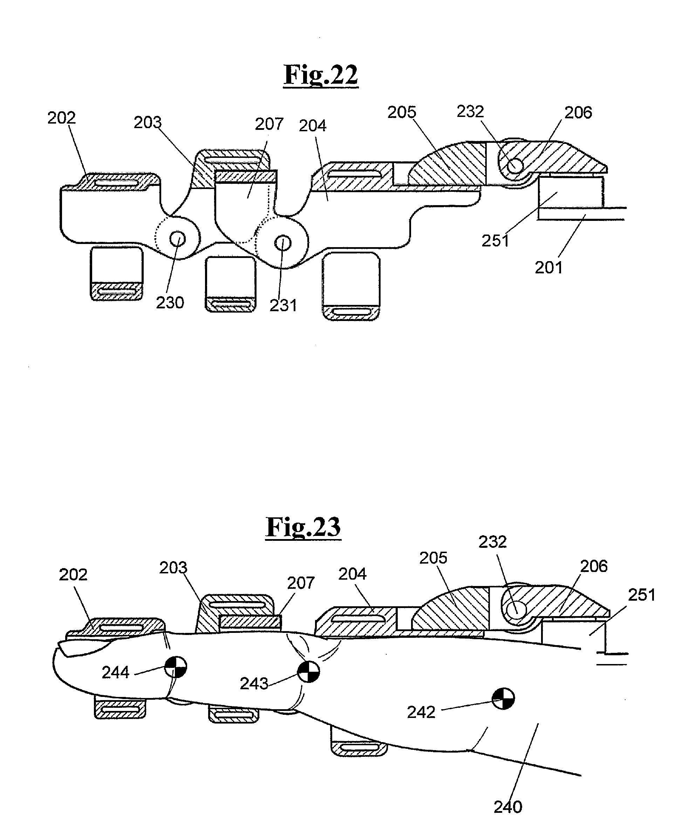

As shown in figures 18 and 19, which are respectively two lateral views of a finger 245 of hand exoskeleton 250, and in figure 23, each shell type elements 202, 203, 204 is coupled to the respective phalanx of the user's finger, then it is operatively integral to the hand.

In a possible embodiment, the shell type elements have fastening elements placed in the lower part of the phalanxes, not integral to the shell type elements but coupled to them by elastic straps or other fastening elements. In this way the shell type elements are coupled to the phalanxes. This fastening elements are not integral to the shell type elements in order to wear on the exoskeleton in an easier and faster way.

Exoskeleton 250 is designed to displace, for each exoskeleton finger, its revolute joints 230, 231 and 232, shown in figures 203, 204 and 207, in coincidence to the respective articulations 244, 242 and 243 of finger 240 of the hand, shown in figure 23.

More precisely, shell type elements 202, 203, 204, 205, 206 and 207 are connected to one another by means of revolute and sliding joints in series so that during the motion of each exoskeleton finger, revolute joints 230, 231 and 232 can move in coincidence to articulations 242, 243 and 244 of finger 240.

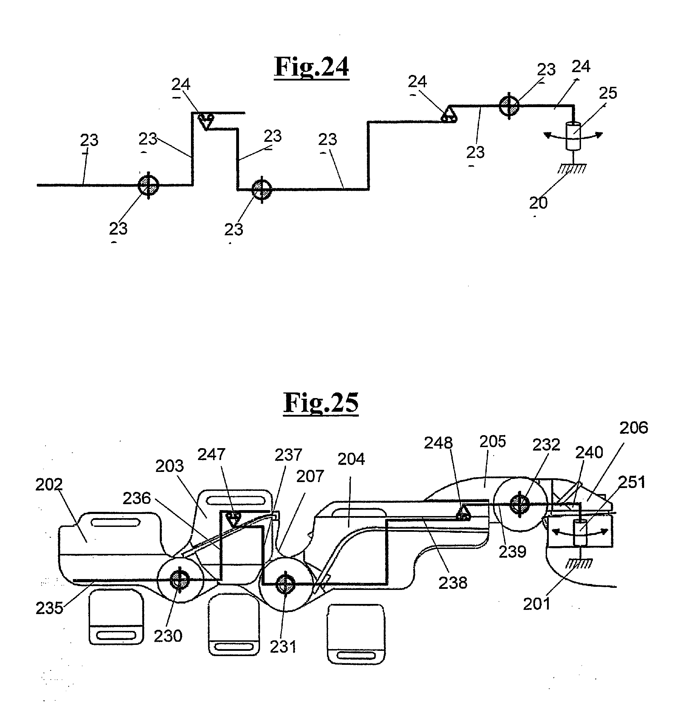

In particular, in the kinematic scheme in figure 9, each finger of the exoskeleton has six joints, where three are actively controlled revolute joints 230, 231 and 232, which correspond to flexion-extension articulations 242 MCP, 243 PIP and 244 DIP of the finger of the hand of the user. The other three joints are passive, and two of them are sliding joints 247 and 248, shown in figures 24 and 25, placed to work respectively between the articulations 244 and 243, and 243 and 242 of the finger; the other is a passive revolute joint 251, shown in figures 18, 19, 22, 23, 24 and 25, and enables the finger to move according to the adduction-abduction DoF substantially perpendicular to revolute joints 230, 231 and 232 that instead are parallel to one another. Therefore, joint 251 enables the exoskeleton finger to move on the lying plane of the hand metacarpus, while the other joints allow the opening-closing movements of the hand by means of flexion-extension movements.

In figures 24 and 25, which show a kinematic scheme in relation to one finger of the exoskeleton, shell type element 202 is depicted with beam member 235, shell type element 203 is schematized with beam member 236, the connecting element 207 is schematized with beam member 237, shell type element 204 is schematized with beam member 238, element 205 is schematized with beam member 239, element 206 is schematized with beam member 240. Beam member 235 is connected with beam member 236 by revolute joint 230, beams 236 and 237 are connected by the guide 241, beams 237 and 238 by revolute joint 231, beams 238 and 239 by guide 242, beams 239 and 240 by revolute joint 232 and beam member 240 is connected to the frame by revolute joint 211.

The two passive sliding joints 247 and 248, shown in figure 24 and 25, together with the inner coating material of shell type elements 202, 203 and 204 of the exoskeleton, allows maintaining a correspondence of the revolute axes, and then maintaining a kinematic compatibility between the movements of the fingers of the user and the movements of exoskeleton 250.

More in detail, sliding joint 247 of figures 24 and 25 is obtained by means of a relative sliding movement between element 203 and element 207 of figures 18, 19, 22, 23, 25, whereas sliding joint 248 of figure 24 and 25 is obtained by the translation between element 204 and element 205 of figures 16-25.

At each actuated revolute joint, i.e. joints 230, 231 and 232, there are two parallel pulleys placed on the lateral sides with respect to the finger, coaxially with each revolute joint and integral to one of the two coupled parts. In particular, as shown in figures 18 and 19, laterally to revolute joint 203 pulleys 210 and 211 are provided that are integral to shell-like element 202, whereas laterally to revolute joint 231 there are pulleys 209 and 212 that are integral to shell-like element 207, and laterally to revolute joint 232 there are pulleys 208 and 213 that are integral to shell-like element 205.

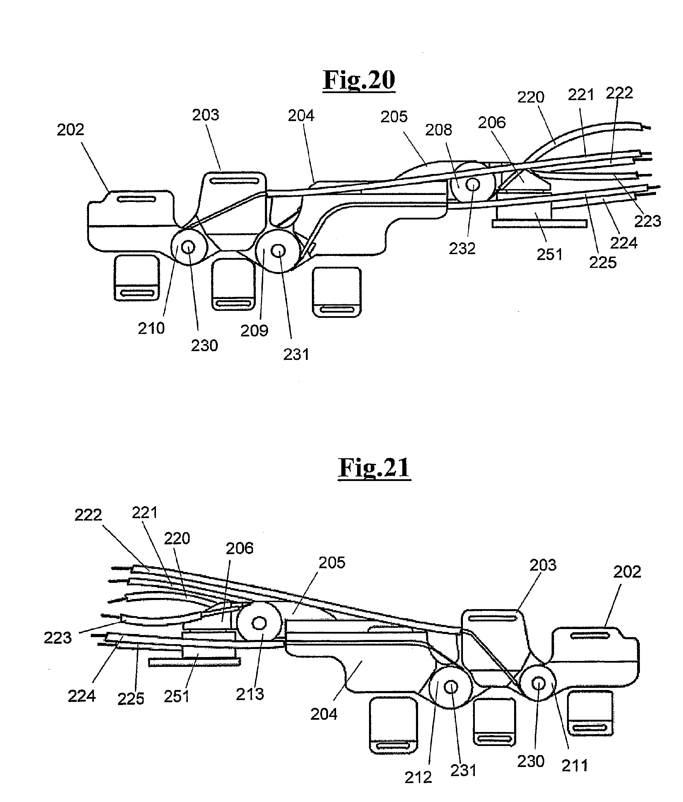

Figures 20 and 21 show the Bowden cables that operate pulleys 208, 209, 210, 211, 212 and 213 that are indicated by numbers 220, 221, 222, 223, 224, 225.

In particular, each active pulley 208, 210, 211, 213, 212, 209 is linked to a respective Bowden cable 220-225, which engages with special grooves 216, 214, 219, 217, 218, 215 machined on shell-like elements 203, 204 that cover the phalanxes and extended on the part 206, up to the actuation system not shown.

The pulleys of couples 210, 211; 209, 212; 208, 213 are respectively integral to each other through the respective shells so that pulling the respective cables the desired agonist-antagonist movement is obtained.

Each Bowden cable 220, 221, 222, 223, 224, 225, if active, causes the rotation of the respective pulley where it is fixed and then causes the flexion or the extension of the corresponding joint.

The actuation system (not shown) of the Bowden cables 220-225 is not assembled on the frame of exoskeleton 250 but is placed in a remote position with respect to the device and is adjusted to be easily moved by a person that is wearing exoskeleton 250. The active joints 230, 231, 232 are equipped with sensors to measure instantaneously both the joint position and the hand posture and the applied torque on each joint by the agonist-antagonist cables.

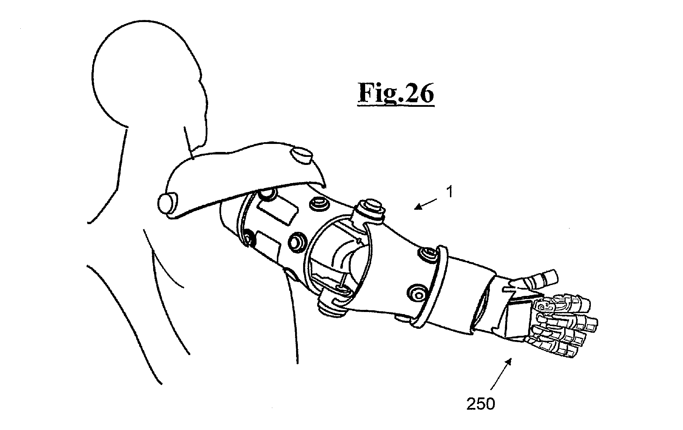

With reference to fig. 26, a combined hand-elbow exoskeleton comprises a hand exoskeleton 250 according to figures 16-25 and an exoskeleton 1 according to figures 1 - 15, mounted at the elbow.

## Claims

1. A wearable mechatronic device for limb rehabilitation, said limb including a proximal element (40) and a distal element (41) connected by a joint, said joint having an instantaneous joint axis, including:
- a proximal stiff bearing structure (2) attachable to said proximal element (40) of said limb and a distal stiff bearing structure (3) attachable to said distal element (41) of said limb, said proximal and distal structures (2,3) pivotally connected about a revolute axis (4);
- means to generate a driving torque between said proximal and distal structures (2,3);
- means to measure the angular position between said proximal and distal structures (2,3);
said device **characterized in that** it includes instantaneous self-aligning means (5) for aligning said revolute axis (4) with said instantaneous joint axis, said instantaneous self-aligning means (5) including means for moving selectively said revolute axis (4) around a point, inside a space defined by two opposite cones (65) having vertex coincident on said point.

2. A mechatronic device, according to claim 1, wherein said self-aligning means (5) includes a couple of opposite adjustable joints (6,7), each adjustable joint (6,7) being formed by a ring and a shaft that is the shaft of said revolute axis (4), said ring and said shaft being blocked from relatively rotating about said revolute axis (4), but being adapted to rotate in a limited way around two axes (66,68, 67,69) perpendicular to said revolute axis (4), such that a conical motion of said shaft with respect to said point is defined, in particular said self-aligning shaft including a torque sensor that is adapted to measure the torque between said proximal structure and said distal structure.

3. A mechatronic device, according to claim 2, wherein each shaft, which is linked between said proximal and distal structures (2,3), pivotally receives a driven pulley integral to the other among said proximal and distal structures (2,3).

4. A mechatronic device, according to claim 2, wherein said opposite adjustable joints (6,7) are respectively connected with a first (11) and a second (12) kinematic chain placed specularly to each other with respect to a longitudinal plane of the limb and connected with said proximal or distal structure (2,3), in particular each kinematic chain (11,12) having in series:
- a rigid link (51,52) having a first end slidingly connected to said adjustable joint (6,7) by means of a circular guide (61,62) and a second end connected to said proximal or distal structure (2,3);
- at least one connecting rod (55) hinged at an end to said rigid link (51,52) close to said second end and at another end to a rocker arm (60);
wherein said rocker arm (60) is centrally hinged to said proximal or distal structure (2,3) and to one end of said connecting rod (55), the other end of said rocker arm (60) being hinged to the connecting rod (55) of the other kinematic chain.

5. A mechatronic device, according to claim 4, wherein said connecting rods (55) have adjustable length and said connecting rods (55) have preferably a ball-and-socket joint connection (56,58 and 57,59) with said rigid link (51,52) or said rocker arm (60).

6. A mechatronic device, according to claim 4, wherein said rigid link (51,52) is L shaped and bent along a substantially cylindrical surface so that it is operatively displaced around the limb.

7. A mechatronic device, according to claim 1, wherein said proximal and distal structures (2,3) have an anatomical substantialy saddle-like shape, with U cross section, to allow to the limb to be housed within them, in particular said structures (2,3) being shaped in order to include mechanical stops to avoid over-extension of the limb.

8. A mechatronic device, according to claim 1, wherein said proximal and distal structures (2,3) have a hollow box-like shape, in particular made of carbon fiber material.

9. A mechatronic device, according to claim 1, wherein said means for generating a driving torque between said first and second structure includes actuating means (23,101,102) in agonist-antagonist configuration, in particular said actuating means in agonist-antagonist configuration comprising:
- a pulley (23) connected with said adjustable shaft, said pulley being integral to said proximal or distal structure (2,3);
- a couple of cables (101,102) that respectively wrap and unwrap on said pulley (23) to operate a rotating motion between said proximal structure (2) and said distal structure (3), said cables (101,102) having an end constrained respectively to a first and a second peripheral connection point of said pulley (23), in particular said cables (101,102) sliding inside of a respective sheath which has an end constrained to said distal structure (3).

10. A mechatronic device, according to claim 9, wherein said actuating means includes a planetary reduction gear having:
- a wrapping pulley for said actuation cables (101,102);
- a sun gear (82) coaxially integral to said wrapping pulley;
- planetary gears (83) that mesh with said sun gear (82);
- a carrier (84) integral to said proximal or distal structure (2, 3) ;
- an internal gear slidingly connected with said shaft of said adjustable joint.

11. A mechatronic device, according to claim 2 and 9 or 10, wherein said torque sensor is integrated on said pulley (23), said pulley having a plurality of spokes, said spokes having at least one strain gauge adapted to transmit force information to a control system, said at least one strain gauge being preferably put in a Wheastone bridge to filter the force components applied by the cables by the pure torque between the structures.

12. A mechatronic device, according to claims 9 or 10, wherein a means is provided to measure the forces applied to each of said actuation cables (101,102), including a load cell (97,98) having a fixed first end and a second end pivotally connected with a deflection pulley (99,100) on which said cable is operatively connected, said cable pressing transversally on said deflection pulley and operating said load cell, said load cell giving as output a signal proportional to force transverse component Fy of said force, said force being obtainable by said force transverse component Fy.

13. A mechatronic device, according to claim 1,
wherein said proximal and distal structures (2,3) comprise an internal shell and an external shell, connected by a coupling means, such as an elastic connection means including at least one elastic bushing (8) with spherical joint to allow a limited relative motion, in particular including a sensor means,
and wherein
said internal shell is operatively in touch with the distal or proximal element of the limb, while said external shell coupled to said internal shell, in particular said proximal or distal internal shell selected from the group comprised of:
- an external layer (72) of thermoforming material, in particular polypropylene;
- an internal padding layer (73) of low thickness expanded polymer material, in particular plastazote or evalux.

14. A mechatronic device, according to claim 1, wherein an actuated hand exoskeleton (250) is further provided for a user's hand, said hand having a metacarpus from which fingers extend formed by proximal phalanxes, medial phalanxes, distal phalanxes and including various articulations among which metacarpal phalangeal articulations (MCP) and abduction-adduction and flexion-extension articulations between said metacarpus and said proximal phalanxes, flexion-extension interphalangeal proximal articulations (PIP) between the proximal phalanxes and the medial phalanxes, and interphalangeal distal articulations (DIP) between the medial phalanxes and the distal phalanxes, said exoskeleton including:
- an external backing element (201) applicable on the metacarpus;
- shell-like elements (202,203,204) applicable on each phalanx, said shell-like elements being connected in series to each other starting from said backing element (201) by means of respective joints (230,231,232);
- means to arrange operatively and automatically said joints in coincidence of each respective articulation of said hand.

15. A mechatronic device in combination with an hand exoskeleton, according to claim 14, wherein said means to arrange said joints comprises:
- three pivotally operated and controlled couplings (230,231,232) corresponding to said flexion-extension MCP, PIP, DIP articulations of said hand;
- two longitudinal sliding couplings (247,248) corresponding to the MCP and PIP articulations of said hand, in particular passive sliding couplings;
- a passive revolute coupling (251) corresponding to said abduction-adduction articulation of said hand,
in particular wherein:
- said sliding coupling (247,248) corresponding to said MCP articulation is placed between said MCP rotating coupling (242) and said PIP rotating coupling (243);
- said sliding coupling (247,248) corresponding to said PIP articulation is placed between said PIP rotating coupling (243) and said DIP rotating coupling (244).

## Patentansprüche

1. Tragbare mechatronische Vorrichtung zur Gliedmaßenrehabilitation, wobei die Gliedmaße ein proximales Element (40) und ein distales Element (41), die durch ein Gelenk verbunden sind, umfasst, wobei das Gelenk eine Momentangelenkachse aufweist, mit:
- einer proximalen steifen Auflagestruktur (2), die an dem proximalen Element (40) der Gliedmaße angebracht werden kann, und einer distalen steifen Auflagestruktur (3), die an dem distalen Element (41) der Gliedmaße angebracht werden kann, wobei die proximale und die distale Struktur (2,3) um eine Drehachse (4) drehbar verbunden sind;
- Mitteln zur Erzeugung eines Antriebsmoments zwischen der proximalen und der distalen Struktur (2,3);
- Mitteln zur Messung der Winkelposition zwischen der proximalen und der distalen Struktur (2,3);
wobei die Vorrichtung **dadurch gekennzeichnet ist, dass** sie Momentan-Selbstausrichtungsmittel (5) zum Ausrichten der Drehachse (4) mit der Momentangelenkachse umfasst, wobei die Momentan-Selbstausrichtungsmittel (5) Mittel zum selektiven Bewegen der Drehachse (4) um einen Punkt herum, innerhalb eines Raums, der durch zwei entgegengesetzte Kegel (65) mit an dem Punkt zusammentreffenden Scheiteln festgelegt ist, umfassen.

2. Mechatronische Vorrichtung nach Anspruch 1, wobei das Selbstausrichtungsmittel (5) ein Paar von entgegengesetzten einstellbaren Gelenken (6,7) umfasst, wobei jedes einstellbare Gelenk (6,7) durch einen Ring und eine Achse, die die Achse der Drehachse (4) ist, gebildet ist, wobei der Ring und die Achse gegenüber einer relativen Rotation um die Drehachse (4) blockiert sind, jedoch derart eingerichtet sind, dass sie sich in beschränkter Weise um zwei, zu der Drehachse (4) senkrechte Achsen (66,68,67,69) derart drehen, dass eine konische Bewegung der Achse in Bezug auf den Punkt festgelegt ist, wobei die Selbstausrichtungsachse insbesondere einen Drehmomentsensor umfasst, der derart eingerichtet ist, dass er das Drehmoment zwischen der proximalen Struktur und der distalen Struktur misst.

3. Mechatronische Vorrichtung nach Anspruch 2, wobei jede Achse, die als Zwischenstück zwischen der proximalen und der distalen Struktur (2,3) eingefugt ist, eine angetriebene Scheibe, die mit der anderen von der proximalen und der distalen Struktur (2,3) integriert ist, drehbar aufnimmt.

4. Mechatronische Vorrichtung nach Anspruch 2, wobei die entgegengesetzten einstellbaren Gelenke (6,7) jeweils mit einer ersten (11) bzw. einer zweiten (12) kinematischen Kette verbunden sind, die in Bezug auf eine Längsebene der Gliedmaße spiegelbildlich zueinander angeordnet sind und mit der proximalen oder der distalen Struktur (2,3) verbunden sind, wobei jede kinematische Kette (11,12) insbesondere aufeinanderfolgend aufweist:
- ein starres Verbindungsstück (51,52) mit einem ersten Ende, das mittels einer kreisförmigen Führung (61,62) mit dem einstellbaren Gelenk (6,7) gleitend verbunden ist, und einem zweiten Ende, das mit der proximalen oder der distalen Struktur (2, ) verbunden ist,
- mindestens einen Verbindungsstab (55), der an einem Ende mit dem starren Verbindungsstück (51,52) nahe an dem zweiten Ende und am anderen Ende mit einem Schwinghebel (60) gelenkig verbunden ist,
wobei der Schwinghebel (60) zentral mit der proximalen oder der distalen Struktur (2,3) und mit einem Ende des Verbindungsstabs (55) gelenkig verbunden ist, wobei das andere Ende des Schwinghebels (60) mit dem Verbindungsstab (55) der anderen kinematischen Kette gelenkig verbunden ist.

5. Mechatronische Vorrichtung nach Anspruch 4, wobei die Verbindungsstäbe (55) eine einstellbare Länge aufweisen und die Verbindungsstäbe (55) vorzugsweise eine Kugelgelenkverbindung (56,58 und 57,59) mit dem starren Verbindungsstück (51,52) oder dem Schwinghebel (60) aufweisen.

6. Mechatronische Vorrichtung nach Anspruch 4, wobei das starre Verbindungsstück (51,52) L-förmig und derart längs einer im Wesentlichen zylindrischen Fläche gebogen ist, dass es im Betrieb um die Gliedmaße herum bewegt ist.

7. Mechatronische Vorrichtung nach Anspruch 1, wobei die proximale und die distale Struktur (2,3) eine anatomische, im Wesentlichen sattelförmige Gestalt mit einem U-förmigen Querschnitt aufweisen, damit die Gliedmaße innerhalb von diesen aufgenommen werden kann, wobei die Strukturen (2,3) insbesondere geformt sind, um mechanische Stopps zu umfassen, um ein Überstrecken der Gliedmaße zu vermeiden.

8. Mechatronische Vorrichtung nach Anspruch 1, wobei die proximale und die distale Struktur (2,3) eine hohlkastenartige Form aufweisen, die insbesondere aus Carbonfasermaterial hergestellt ist.

9. Mechatronische Vorrichtung nach Anspruch 1, wobei das Mittel zum Erzeugen eines Antriebsmoments zwischen der ersten und der zweiten Struktur Betätigungsmittel (23,101,102) in Agonist/Antagonist-Konfiguration enthält, wobei die Betätigungsmittel in Agonist/nntagonist-Konfiguration umfassen:
- eine Riemenscheibe (23), die mit der einstellbaren Achse verbunden ist, wobei die Riemenscheibe mit der proximalen oder der distalen Struktur (2,3) integriert ist;
- ein Paar von Seilen (101,102), die sich auf der Riemenscheibe (23) auf- bzw. abwickeln, wobei eine Drehbewegung zwischen der proximalen Struktur (2) und der distalen Struktur (3) bewirkt wird, wobei die Seile (101,102) ein Ende jeweils an einem ersten bzw. zweiten peripheren Verbindungspunkt der Riemenscheibe (23) befestigt haben, wobei die Seile (101,102) insbesondere im Inneren einer jeweiligen Hülle gleiten, die ein Ende an der distalen Struktur (3) festgelegt hat.

10. Mechatronische Vorrichtung nach Anspruch 9, wobei das Betätigungsmittel ein Planetenreduktionsgetriebe umfasst mit:
- einer Aufwickelscheibe für die Betätigungsseile (101,102),
- einem Sonnenrad (82), das mit der Aufwickelscheibe koaxial integriert ist,
- Planetenrädern (83), die mit dem Sonnenrad (82) in Eingriff stehen;
- einem Träger (84), der mit der proximalen oder der distalen Struktur (2,3) integriert ist,
- einem Innenrad, das gleitend mit der Achse des einstellbaren Gelenks verbunden ist.

11. Mechatronische Vorrichtung nach Anspruch 2 und 9 oder 10, wobei der Drehmomentsensor an der Riemenscheibe (23) integriert ist, wobei die Riemenscheibe eine Mehrzahl von Speichen aufweist, wobei die Speichen mindestens einen Belastungsmesser aufweisen, der zum Übermitteln von Kraftinformationen an ein Steuersystem eingerichtet ist, wobei der mindestens eine Belastungsmesser vorzugsweise in eine Wheatstone-Brücke eingebaut ist, um die durch das reine Drehmoment zwischen den Strukturen durch die Seile ausgeübten Kraftkomponenten zu filtern.

12. Mechatronische Vorrichtung nach Anspruch 9 oder 10, wobei ein Mittel zum Messen der Kräfte, die auf jedes der Betätigungsseile (101,102) ausgeübt werden, vorgesehen ist, das eine Lastzelle (97,98) mit einem feststehenden ersten Ende und einem zweiten Ende, das schwenkbar verbunden ist mit einer Ablenkriemenscheibe (99,100), an der das Seil operativ verbunden ist, umfasst, wobei das Seil quer auf die Ablenkriemenscheibe drückt und auf die Lastzelle wirkt, wobei die Lastzelle als Ausgabe ein zur Kraftquerkomponente Fy der Kraft proportionales Signal ausgibt, wobei die Kraft durch die Kraftquerkomponente Fy erhalten werden kann.

13. Mechatronische Vorrichtung nach Anspruch 1, wobei die proximale und die distale Struktur (2,3) eine Innenhülse und eine Außenhülse umfassen, die durch ein Kopplungsmittel, beispielsweise ein elastisches Verbindungsmittel, verbunden sind, das mindestens eine elastische Lagerbuchse (8) mit einem kugelförmigen Verbindungsstück umfasst, um eine beschränkte relative Bewegung zu ermöglichen, und insbesondere ein Sensormittel umfasst,
und wobei
die Innenhülse operativ in Berührung mit dem distalen oder proximalen Element der Gliedmaße ist, während die *Außenhülse an die Innenhülse gekoppelt ist,
wobei die proximale oder die distale Innenhülse insbesondere aus der Gruppe von:
- einer äußeren Schicht (72) aus einem Wärmeformmaterial, insbesondere Polypropylen;
- einer inneren Polsterschicht (73) aus einem geschäumten Polymermaterial geringer Dicke, insbesondere Plastazote oder Evalux, ausgewählt ist.

14. Mechatronische Vorrichtung nach Anspruch 1, wobei ferner ein betätigbares Handexoskelett (250) für eine Hand eines Nutzers vorgesehen ist, wobei die Hand eine Mittelhand aufweist, von der sich Finger erstrecken, die aus Fingergrundgliedern, Fingermittelgliedern, Fingerendgliedern gebildet sind und verschiedene Gelenke umfassen, die Fingergrundgelenke (Articulationes metacarpophalangeales, MCP) und Abduktion-Adduktion- und flexion-Extension-Gelenke zwischen der Mittelhand und den Fingergrundgliedern, Flexion-Extension-Fingermittelgelenke (Articulationes interphalangeales proximales, PTP) zwischen den Fingergrundgliedern und den Fingermittelgliedern und Fingerendgelenke (Articulationes interphalangeales distales, DIP) zwischen den Fingermittelgliedern und den Fingerendgliedern umfassen, wobei das Exoskelett umfasst:
- ein äußeres Stützelement (201), das auf der Mittelhand anzubringen ist,
- hülsenartige Elemente (202,203,204), die auf jedem Fingerglied anzubringen sind, wobei die hülsenartigen Elemente ausgehend von dem Stützelement (201) mittels jeweiliger Gelenke (230,231,232) aufeinanderfolgend miteinander verbunden sind,
- Mittel zum operativen und automatischen Anordnen der Gelenke in Übereinstimmung mit jeder entsprechenden Bewegung der Hand.

15. Mechatronische Vorrichtung in Kombination mit einem Handexoskelett nach Anspruch 14, wobei das Mittel zum Anordnen der Gelenke umfasst:
- drei drehbar arbeitende und gesteuerte Kopplungen (230,231,232) entsprechend den Flexion-Extension-MCP-, -PIP-, -DIP-Gelenken der Hand,
- zwei Längsgleitkopplungen (247,248) entsprechend den MCP- und PTP-Gelenken der Hand, insbesondere passive Gleitkopplungen,
- eine passive Drehkupplung (251) entsprechend dem Abduktion-Adduktion-Gelenk der Hand,
wobei insbesondere:
- die Gleitkopplung (247,248) entsprechend dem MCP-Gelenk zwischen der MCP-Rotationskopplung (242) und der PIP-Rotationskopplung (243) platziert ist;
- die Gleitkupplung (247,248) entsprechend dem PIP-Gelenk zwischen der PIP-Rotationskopplung (243) und der DIP-Rotationskopplung (244) platziert ist.

## Revendications

1. Dispositif mécatronique portable pour la rééducation d'un membre, ledit membre comprenant un élément proximal (40) et un élément distal (41) raccordés par une articulation, ladite articulation ayant un axe d'articulation instantané, ledit dispositif comprenant :
- une structure de support rigide proximale (2) pouvant être fixée au dit élément proximal (40) dudit membre et une structure de support rigide distale (3) pouvant être fixée au dit élément distal (41) dudit membre, lesdites structures proximale et distale (2, 3) étant raccordées de manière pivotante autour d'un axe de rotation (4) ;
- un moyen de génération d'un couple d'entraînement entre lesdites structures proximale et distale (2, 3) ;
- un moyen de mesure de la position angulaire entre lesdites structures proximale et distale (2, 3) ;
ledit dispositif étant **caractérisé en ce qu'**il comprend un moyen d'aulo-alignement instantané (5) pour aligner ledit axe de rotation (4) sur ledit axe d'articulation instantané, ledit moyen d'auto-alignement instantané (5) comprenant un moyen de déplacement sélectif dudit axe de rotation (4) autour d'un point, à l'intérieur d'un espace défini par deux cônes opposés (65) ayant un vertex coïncident sur ledit point.

2. Dispositif mécatronique, selon la revendication 1, dans lequel ledit moyen d'auto-alignement (5) comprend un couple d'articulations ajustables opposées (6, 7), chaque articulation ajustable (6, 7) étant formée par un anneau et un arbre qui est l'arbre dudit axe de rotation (4), ledit anneau et ledit arbre ne pouvant pas être entraînés en rotation relative autour dudit axe de rotation (4), mais étant adaptés à être entraînés en rotation de manière limitée autour de deux axes (66, 68, 67, 69) perpendiculaires au dit axe de rotation (4), de telle sorte qu'un mouvement conique dudit arbre relativement au dit point est défini, ledit arbre d'auto-alignement comprenant en particulier un capteur de couple qui est conçu pour mesurer le couple entre ladite structure proximale et ladite structure distale.

3. Dispositif mécatronique, selon la revendication 2, dans lequel chaque arbre, qui est lié à l'une de ladite structure proximale et de ladite structure distale (2, 3), reçoit de manière pivotante une poulie menée formant partie intégrante de l'autre de ladite structure proximale et de ladite structure distale (2, 3).

4. Dispositif mécatronique, selon la revendication 2, dans lequel lesdites articulations ajustables opposées (6, 7) sont respectivement raccordées à des première (11) et seconde (12) chaînes cinématiques placées de manière spéculaire l'une par rapport à l'autre relativement à un plan longitudinal du membre et raccordées à ladite structure proximale ou distale (2, 3), en particulier chaque chaîne cinématique (11, 12) ayant en série :
- un lien rigide (51, 52) ayant une première extrémité raccordée de manière coulissante à ladite articulation ajustable (6, 7) au moyen d'un guide circulaire (61, 62) et une seconde extrémité raccordée à ladite structure proximale ou distale (2, 3) ;
- au moins une tige de raccordement (55) articulée au niveau d'une extrémité au dit lien rigide (51, 52) à proximité de ladite seconde extrémité et au niveau d'une autre extrémité à un bras oscillant (60) ;
dans lequel ledit bras oscillant (60) est centralement articulé à ladite structure proximale ou distale (2, 3) et à une extrémité de ladite tige de raccordement (55), l'autre extrémité dudit bras oscillant (60) étant articulée à ladite tige de raccordement (55) de l'autre chaîne cinématique.

5. Dispositif mécatronique, selon la revendication 4, dans lequel lesdites tiges de raccordement (55) ont une longueur ajustable et lesdites tiges de raccordement (55) sont de préférence raccordées par articulation à rotule (56, 58 et 57, 59) au dit lien rigide (51, 52) ou au dit bras oscillant (60).

6. Dispositif mécatronique, selon la revendication 4, dans lequel ledit lien rigide (51, 52) est en forme de L et incurvé le long d'une surface sensiblement cylindrique de telle sorte qu'il est fonctionnellement déplacé autour du membre.

7. Dispositif mécatronique, selon la revendication 1, dans lequel lesdites structures proximale et distale (2, 3) ont une forme de type selle sensiblement anatomique, avec une section transversale en U, pour permettre que le membre soit logé à l'intérieur de celles-ci, lesdites structures (2, 3) étant en particulier formées de manière à inclure des arrêts mécaniques pour éviter la sur-extension du membre.

8. Dispositif mécatronique, selon la revendication 1, dans lequel lesdites structures proximale et distale (2, 3) ont une forme de type boite creuse, et sont en particulier fabriquées en un matériau de fibre de carbone.

9. Dispositif mécatronique, selon la revendication 1, dans lequel lesdits moyens de génération d'un couple d'entraînement entre lesdites première et seconde structures comprennent des moyens d'actionnement (23, 101, 102) dans une configuration agoniste-antagoniste, ledit moyen d'actionnement en configuration agoniste-antagoniste comprenant en particulier :
- une poulie (23) raccordée au dit arbre ajustable, ladite pouline formant partie intégrante de ladite structure proximale ou distale (2, 3) ;
- un couple de câbles (101, 102) qui respectivement s'enroulent et se déroulent sur ladite poulie (23) pour opérer un mouvement de rotation entre ladite structure proximale (2) et ladite structure distale (3), lesdits câbles (101, 102) ayant une extrémité contrainte respectivement à un premier et un second point de raccordement périphérique de ladite poulie (23), en particulier lesdits câbles (101, 102) coulissant à l'intérieur d'une gaine respective qui a une extrémité contrainte à ladite structure distale (3).

10. Dispositif mécatronique, selon la revendication 9, dans lequel ledit moyen d'actionnement comprend un engrenage de réduction planétaire ayant :
- une poulie d'enroulement pour lesdits câbles d'actionnement (101, 102) ;
- un planétaire (82) formant coaxialement partie intégrante de ladite poulie d'enroulement ;
- des engrenages planétaires (83) qui s'engrènent avec ledit planétaire (82) ;
- un vecteur (84) faisant partie intégrante de ladite structure proximale ou distale (2, 3) ;
- un engrenage interne raccordé de manière coulissante à ladite tige de ladite articulation ajustable.

11. Dispositif mécatronique, selon les revendications 2 et 9 ou 10, dans lequel ledit capteur de couple est intégré sur ladite poulie (23), ladite poulie ayant une pluralité de rayons, lesdits rayons ayant au moins une jauge de contrainte adaptée à transmettre des informations de force à un système de commande, ladite ou lesdites jauges de contrainte étant de préférence placées dans un pont de Wheatstone pour filtrer les composantes de force appliquées par les câbles par le couple pur entre les structures.

12. Dispositif mécatronique, selon la revendication 9 ou 10, dans lequel un moyen est fourni pour mesurer les forces appliquées à chacun desdits câbles d'actionnement (101, 102), comprenant une cellule de charge (97, 98) ayant une première extrémité fixée et une seconde extrémité raccordée de manière pivotante à une poulie de déviation (99, 100) sur laquelle ledit câble est raccordé de manière opérationnelle, ledit câble appuyant transversalement sur ladite poulie de déviation et faisant fonctionner ladite cellule de charge, ladite cellule de charge donnant comme sortie un signal proportionnel à la composante transversale de force Fy de ladite force, ladite force pouvant être obtenue par ladite composante transversale de force Fy.

13. Dispositif mécatronique, selon la revendication 1,
dans lequel lesdites structures proximale et distale (2, 3) comprennent une coque interne et une coque externe, raccordées par un moyen de couplage, tel qu'un moyen de raccordement élastique comprenant au moins une bague élastique (8) avec une articulation sphérique pour permettre un mouvement relatif limité, comprenant en particulier un moyen de capteur,
et dans lequel
ladite coque interne est en contact fonctionnel avec ledit élément distal ou proximal du membre, alors que ladite coque externe est couplée à ladite coque interne, en particulier ladite coque interne proximale ou distale est sélectionnée dans le groupe comprenant :
- une couche externe (72) de matériau de thermoformage, en particulier de polypropylène ;
- une couche de remplissage interne (73) en un matériau polymère expansé de faible épaisseur, en particulier en plastazote ou evalux.

14. Dispositif mécatronique, selon la revendication 1, dans lequel un exosquelette de main actionné (250) est en outre fourni pour une main d'utilisateur, ladite main ayant un métacarpe à partir duquel s'étendent des doigts formés par des phalanges proximales, des phalanges médiales, des phalanges distales et comprenant différentes articulations parmi lesquelles des articulations métacarpo-phalangiennes (MCP) et des articulations d'abduction-adduction et de flexion-extension entre ledit métacarpe et lesdites phalanges proximales, des articulations interphalangiennes proximales de flexion extension (PIP) entre les phalanges proximales et les phalanges médiales, et des articulations interphalangiennes distales (DIP) entre les phalanges médiales et les phalanges distales, ledit exosquelette comprenant :
- un élément de support externe (201) applicable sur le métacarpe ;
- des éléments de type coque (202, 203, 204) applicables sur chaque phalange, lesdits éléments de type coque étant raccordés en série les uns aux autre à partir dudit élément de support (201) au moyen d'articulations respectives (230, 231, 232) ;
- un moyen pour agencer de manière fonctionnelle et automatique lesdites articulations en coïncidence avec chaque articulation respective de ladite main.

15. Dispositif mécatronique combiné à un exosquelette de main, selon la revendication 14, dans lequel ledit moyen destiné à agencer lesdites articulations comprend :
- trois couplages commandés et opérés de manière pivotante (230, 231, 232) correspondant auxdites articulations DIP, PIP, MCP de flexion-extension de ladite main ;
- deux couplages coulissants longitudinaux (247, 248) correspondant aux articulations MCP et PIP de ladite main, en particulier des couplages coulissants passifs ;
- un couplage de rotation passif (251) correspondant ladite articulation d'abduction-adduction de ladite main,
en particulier dans lequel :
- ledit couplage coulissant (247, 248) correspondant à ladite articulation MCP est placé entre ledit couplage de rotation MCP (242) et ledit couplage de rotation PIP (243) ;
- ledit couplage coulissant (247, 248) correspondant à ladite articulation PIP est placé entre ledit couplage de rotation PIP (243) et ledit couplage de rotation DIP (244).
